# EUROPEAN PATENT SPECIFICATION

(11) **EP 4 639 138 B1**
(45) Date of publication and mention of the grant of the patent: **18.11.2026**
(21) Application number: 23837679.2
(22) Date of filing: 22.12.2023
(51) Int. Cl.: G01N 15/14, G06T 7/73, G01N 15/10, G01N 15/1429, G01N 15/1434, G01N 15/1433

(54) **METHOD AND SYSTEM FOR DETERMINING SPATIAL LOCATION OF BIOLOGICAL COMPONENTS IN A SAMPLE**
VERFAHREN UND SYSTEM ZUR BESTIMMUNG DER RÄUMLICHEN POSITION VON BIOLOGISCHEN KOMPONENTEN IN EINER PROBE
PROCÉDÉ ET SYSTÈME DE DÉTERMINATION DE L'EMPLACEMENT SPATIAL DE COMPOSANTS BIOLOGIQUES DANS UN ÉCHANTILLON

(30) Priority: 22.12.2022 EP 22383262
(43) Date of publication of application: 29.10.2025
(73) Proprietor: European Molecular Biology Laboratory, 69117 Heidelberg (DE); Fundació Centre de Regulació Genòmica, 08003 Barcelona (ES); Institució Catalana De Recerca I Estudis Avançats, 08010 Barcelona (ES)
(72) Inventor: COTTERELL, James, 08003 Barcelona (ES); SHARPE, James, 08003 Barcelona (ES); SWOGER, James, 08003 Barcelona (ES); ROBERT-MORENO, Alexandre, 08003 Barcelona (ES)
(74) Representative: Sonnenberg Harrison Partnerschaft mbB
(86) International application number: PCT/EP2023/087544
(87) International publication number: WO 2024/133856

(56) References cited:
- DE VRIES NATASJA L. ET AL: "Unraveling the Complexity of the Cancer Microenvironment With Multidimensional Genomic and Cytometric Technologies", FRONTIERS IN ONCOLOGY, vol. 10, 23 July 2020 (2020-07-23), CH, XP093118617, ISSN: 2234-943X, DOI: 10.3389/fonc.2020.01254
- LONGO SOPHIA K ET AL: "Integrating single-cell and spatial transcriptomics to elucidate intercellular tissue dynamics", NATURE REVIEWS GENETICS, NATURE PUBLISHING GROUP, GB, vol. 22, no. 10, 18 June 2021 (2021-06-18), pages 627 - 644, XP037563073, ISSN: 1471-0056, [retrieved on 20210618], DOI: 10.1038/S41576-021-00370-8
- RIECKHER MATTHIAS: "Light Sheet Microscopy to Measure Protein Dynamics", JOURNAL OF CELLULAR PHYSIOLOGY, vol. 232, no. 1, 24 June 2016 (2016-06-24), US, pages 27 - 35, XP093118618, ISSN: 0021-9541, DOI: 10.1002/jcp.25451
- KWON JIWOONG ET AL: "Bleaching-Resistant Super-Resolution Fluorescence Microscopy", ADVANCED SCIENCE, vol. 9, no. 9, 27 January 2022 (2022-01-27), pages 2101817 - 1, XP093118619, ISSN: 2198-3844, DOI: 10.1002/advs.202101817

## Description

### Reference to other applications

The present application claims priority of European Patent application EP 22383262.7, filed on 22 December 2022.

### Field of the Invention

The present invention relates to a method and system of determining spatial location of biological components in a biological sample.

### Background of the invention

In the field of tissue biology and cancer, it is desirable to know information about the spatiotemporal distribution of biological components of cell machinery, genome status, metabolic products and gene activity, in order to understand tissue biology and cancer. To that end, various different spatial omics techniques have been invented to try and measure genomic sequence, protein levels, epigenetic marker levels, metabolic product levels and gene expression levels in different regions of a biological sample.

It is known to use markers which have a known spatial distribution in the biological sample. In the context of spatial transcriptomics, those markers are levels of gene products which can be read out using in situ hybridization or the Hybridization Chain Reaction (HCR), for example. Each region of the sample has a unique signature of marker gene levels that unambiguously defines the region if enough marker genes are used. The biological sample can be dissociated and processed by standard single cell approaches and then the measured levels of those marker genes used to determine location of the cells in the biological tissues.

Such methods require *a priori* information about how those marker genes are distributed in the biological tissues. However, mutants (including cancer samples) do not have such *a priori* information available. Therefore, methods have been developed that require no *a priori* information about distribution of the marker genes in the biological tissues of interest.

*Non-a priori* information methods have focused on taking 2D sections of the biological tissues and placing the 2D sections on slides. To do spatial transcriptomics, for example, the slides have been engineered to have oligonucleotide signatures in known positions and the first step of RNASeq library preparation performed on the slide before digestion of the biological tissues and pooling. The signature can then be used to determine where each read in the resulting RNAseq data originated in the biological sample. Alternatively, the slide is serially stained with probes for specific combinations of gene products and imaged to determine the levels of those gene products over space.

All of these techniques are however inherently 2-dimensional (2D) and have a poor capture depth (i.e., number of unique mRNAs read per cell) among other drawbacks.

For example, the publication entitled "Unraveling the Complexity of the Cancer Microenvironment With Multidimensional Genomic and Cytometric Technologies.", De Vries Natasja L et al, in FRONTIERS IN ONCOLOGY 2020, vol. 10, 2020, page 1254, teaches sectioning the biological tissues and labelling of patterns that are endogenous to the unperturbed tissue. Single-cell data are obtained by flow and mass cytometry that make use of antibodies coupled to fluorochromes or heavy metal isotopes, respectively, for the immunodetection of dissociated cells. For single-cell RNA-sequencing, antibodies coupled to oligonucleotides can be used to simultaneously retrieve information on protein and RNA expression of single cells." which then can be placed in space by integrating this data with spatial omics information.

The publication entitled "Integrating single-cell and spatial transcriptomics to elucidate intercellular tissue dynamics", Longo Sophia K et al, in Nature Reviews Genetics, vol. 22, no. 10, 18 June 2021 describes Niche-seq technique.

The two above publications propose obtaining data from two different techniques: 1) a spatial omic approach and 2) a single cell omics approach. This means that different cells are sampled in each of the techniques. A deconvolution or mapping of the cells used in the single cell omics approach to the cells used in the spatial omics approach is required. This approach introduces errors, since the mapping is not one-to-one and there is no guarantee that the same cell types have been sampled by both techniques.

It is an object of the present disclosure to provide a method for obtaining spatial information of biological components in a biological sample without *a priori* information.

### Brief Summary of the invention

To this end, the present invention proposes a method according to claim 1 and a system according to claim 16.

The method for determining spatial location of one or more biological components in a sample comprises optically encoding the biological components in the sample with a plurality of fluorophores to create a combination of active fluorophore concentrations indicative of the spatial location of the one or more biological components; dissociating the biological components from the sample to create dissociated biological components of the biological components; and mapping the fluorescence measured from the dissociated biological components, to the spatial location of the biological components in the non-dissociated sample.

The present invention therefore proposes a novel approach to determine spatial location of the one or more biological components, e.g. cells in a sample. The present invention proposes to optically encode with a unique signature the different components of the sample, e.g. the cells. By measuring and analysing the fluorescence after dissociation, it is possible to obtain the localisation of the optically encoded components before dissociation of the sample.

The step of optically encoding the biological components in the sample with a plurality of fluorophores to create a combination of active fluorophore concentrations indicative of the spatial location of the one or more biological components is a step of imposing a coordinate system on the sample. The active fluorophore concentrations represent spatial coordinates in said coordinate system. Each component of the sample has therefore unique sample coordinates or spatial data in said system represented by the combination of active fluorophore concentrations.

For a biological component, the combination of active fluorophore concentrations is therefore a unique signature, and this unique signature identifies the position of the biological components in the sample.
With other words, the set of active fluorophore concentrations is a direct proxy for spatial position of the biological component. Hence, single cell omics data and spatial data are generated from the exact same biological components.

In the present invention on the other hand, it is possible to have omics data of a biological component whose spatial location in the sample is explicitly known.

This is advantageous over prior art methods, in which data are integrated from two different techniques: a spatial omics technique and a single cell omics technique. In the prior art, different cells are sampled in each of the techniques, because, once a cell is used for one technique, it cannot be used for the other technique. The prior art methods therefore have a deconvolution or mapping of the cells used in the cell omics technique to the cells used in the spatial omics technique. In the present invention, both the spatial information and the omics information for a biological component are derived from the same biological component. There is no need to perform any form of mapping from the spatial omics approach to the single cell omics approach, thereby preventing the introduction of errors. In addition, less tissue needs to be used.

The encoded coordinate system is preferably an orthogonal cartesian coordinate system.

In one aspect, the biological sample chemically treated to render it optically transparent.

The optical encoding of the biological components may comprise staining the sample with said plurality of fluorophores and altering active fluorophore concentrations of ones of the plurality of fluorophores in the sample after the staining. By altering the active fluorophore concentrations, a unique signature for each component can be generated. A coordinate system is imposed on the sample. For each biological component, the unique signature is the set of sample coordinates in the coordinate system, hence spatial data identifying the location of the biological component in the sample.

In one aspect, the method comprises creating a plurality of gradients of active fluorophore concentrations in the sample, in particular wherein the plurality of gradients of active fluorophore concentrations are orthogonal to each other. Having three orthogonal gradient allows marking three spatial directions in an orthogonal cartesian coordinate system.

The altering of the active fluorophore concentrations may be carried out by one of photobleaching, photoactivation or photo-switching. It is possible to generate gradients that are constant over the sample.

The altering can be carried out by photobleaching, photo-activating, or phot-switching using a light sheet with differential periods of exposure of sections of the sample to the light sheet.

In another aspect, the ratios of the fluorophore concentrations across the biological object are constant between the components, prior to creating the plurality of gradients.

In one embodiment of the invention it is envisaged that at least one, preferably two, of the plurality of fluorophores is covalently attached to an oligonucleotide.

The optical encoding can be carried out by one or more different fluorophores, wherein the different fluorophores have different excitation, activating, or switching spectra. The different emission spectra define independent fluorescence channels.

Additional fluorophores can be used as control fluorophores. It is provided that the control fluorophores remain unaltered, whilst the other fluorophores are altered for optically encoding the biological components in the sample. The step of normalising the measured fluorescence levels of the altered fluorophores with respect to the fluorescence levels of the control fluorophores can be provided. Having a control fluorophore helps improving the accuracy of the determination, in particular if the initial staining before alteration of the fluorophores is not uniform in the sample.

The method may comprise utilizing a calibration sample where the change in the concentration of each of the active fluorophores is recorded by imaging the calibration sample as each fluorophore is altered with increasing exposure in a different tissue slice. The alteration parameters can be determined for altering the sample from the resulting alteration calibration graph, to determine the exposure for each position along the axis to achieve a desired gradient in the sample.

The method may comprise mapping the measured fluorescence levels of the different fluorophores of the dissociated biological components to the physical space in the sample before dissociation, such that each colour combination measured after the dissociating is assigned to a probable location in physical space.

In one aspect, the mapping of measured levels of fluorescence of dissociated biological components to the physical space comprises imaging the biological sample before dissociation and mapping measured colour space of the dissociated biological components to the measured colour space in the system used to image the biological sample before dissociation, by means of a histogram matching algorithm for each fluorescence channel.

Preferably, single fluorophore stained control samples are used to determine cross-talk between pair-wise combinations of channels, in both the system used to image the sample before dissociation and the system used to measure the signals of the biological components after dissociation, to correct channel data in both systems before histogram matching each channel between the two instruments.

The sample can be reconstructed from the measured fluorescence.

The present invention also proposes a system for determining spatial location of one or more biological components in a sample. The system comprises an optical encoding unit provided for optically encoding the biological components in the sample with a plurality of fluorophores to create a combination of active fluorophore concentration indicative of the spatial location of the one or more biological components; an optical measuring system to measure fluorescence after dissociation, comprising one of a fluorescent activated cell sorter or analyse, a microfluidic device; and a mapping unit, configured to mapping the fluorescence measured from the dissociated biological components, to the spatial location of the biological components in the non-dissociated sample.

The optical encoding can be done by staining and altering the biological components in the sample with a plurality of fluorophores to create a combination of active fluorophore concentration indicative of the spatial location of the one or more biological components.

The optical encoding unit can comprise an imaging unit to alter the spatial distribution of plurality of fluorophore concentrations and measure the spatial distribution of the plurality of fluorophore concentrations after alteration, in particular wherein the imaging unit comprises a Selective Plane Illumination Microscope for imaging the sample before dissociation.

The present document therefore describes a method with potentially equal resolution in all dimensions. Attempting to do 3-dimensional omics methods with slide-based approaches has various problems/issues including aligning consecutive slides, loss of spatial resolution and information in cutting edges, non-equal resolution along the different axes, labour time, and cost.

The method set out in this document does not require combining a spatial omics data with scRNASeq data. Therefore, the risk of errors is diminished since the errors are not introduced by inaccurate mapping of single cell RNASeq to spatial information.

The method potentially allows a greater capture depth than slide-based approaches. Indeed, the method and system can be used like a module in a methods pipeline. The method can be performed directly upstream of single cell RNASeq, which means that the capture depth approaches that of scRNASeq. This is a much higher capture depth than slide-based spatial omics approaches of the prior art that need to supplement the spatial information from the spatial omics approach with single cell RNA sequencing data to get the equivalent capture depth.

Finally, the method allows obtaining both the spatial information of a spatial omics approach in combination with the capture depth of single cell RNASeq in a single technique. Therefore, less biological tissue needs to be used. This is particularly important for contexts where tissue is scarce (tumour biopsies for example).

### Description of the figures

Fig. 1 shows an overview of a system according to one aspect of the invention
Fig.2 shows an overview of a method according to one aspect of the invention.
Fig. 3 shows a method of optically encoding according to one aspect of the invention.
Fig. 4 shows a method of optical decoding according to one aspect of the invention.
Fig. 5 shows an example.

### Detailed description of the invention

The invention will now be described on the basis of the drawings.

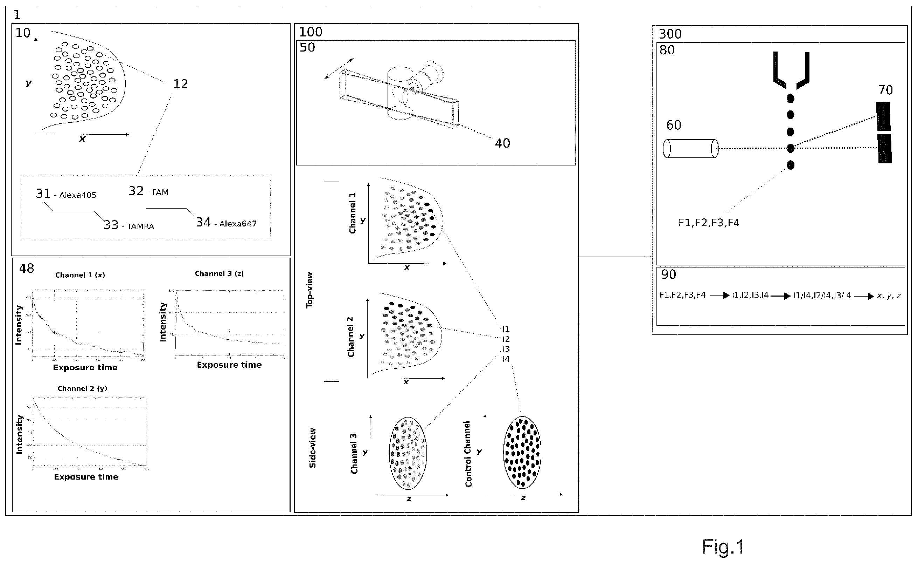

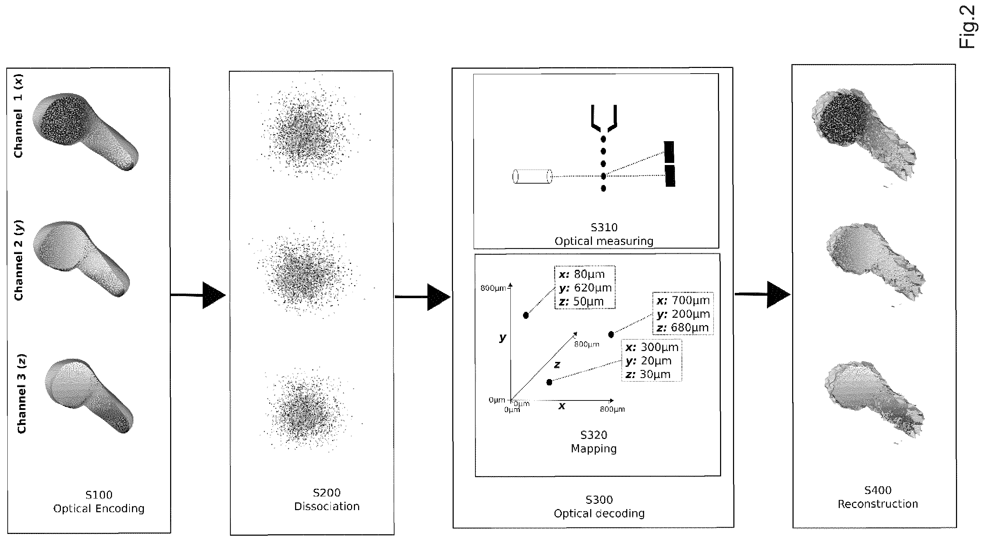

Fig 1 shows an overview of a system 1 for determining spatial location of a plurality of biological components in a biological sample 10, and Fig. 2 an overview of method for determining spatial location of biological components in the biological sample 10.

The biological sample 10 comprises biological components 12 and it is desirable to know information of the spatial distribution of said biological components 12 in the sample 10.

In the present disclosure, an example of biological components 12 are cells of the biological sample 10.

The method comprises a step of optically encoding S100 the biological components 12 in the biological sample 10 to create a unique optical signature for each of the biological components 12 of the biological sample 10. The biological components 12 are optically encoded in the biological sample 10 with a plurality of fluorophores 31, 32, 33, by creating a combination of active fluorophore concentration indicative of a spatial location of the biological components in the biological sample 10. A unique signature indicative of the spatial location is obtained by a combination of the different active fluorophores and this unique signature acts as an optical signature.

Each biological component of the biological sample 10 has a set of sample coordinates or spatial data represented by the combination of active fluorophore concentrations. This set of sample coordinates is the optical signature.

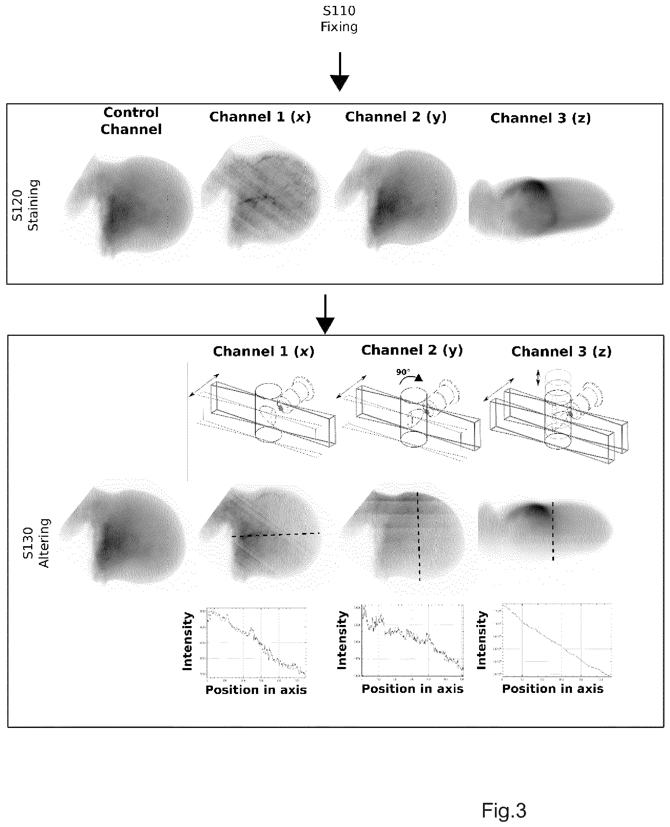

The optical encoding will be described in more detail later with reference to figure 3.

After the optically encoding, the method for determining spatial location of biological components 12 in a biological sample 10 comprises the step of disassociating S200 the biological components 12 from the biological sample 10 to create dissociated biological components 12 of the biological components 12.

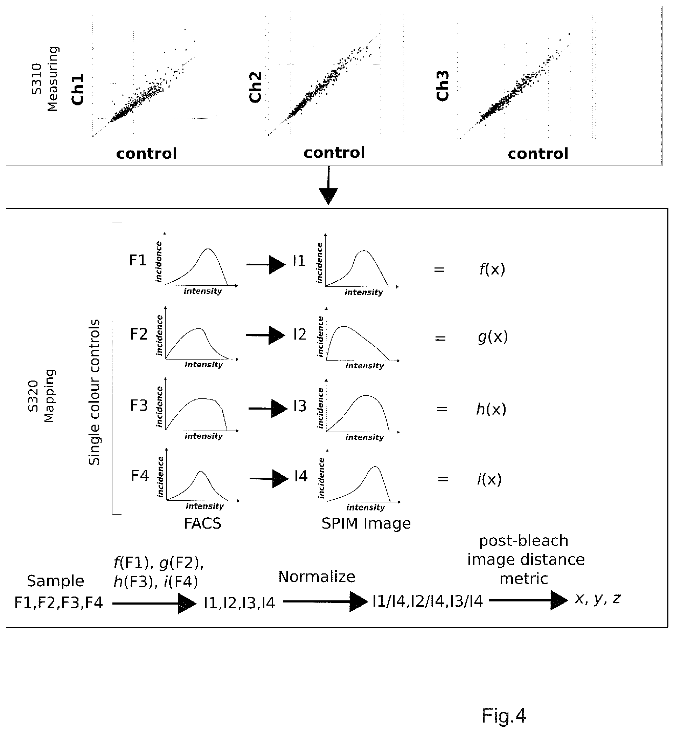

After the dissociation, the measured fluorescence from the dissociated biological components 12 is mapped to the spatial location of the biological components 12 in the non-dissociated sample at step S320, to obtain the original location of the biological components in the non-dissociated sample 10. The mapping will be described later with reference to figure 4.

Measuring the fluorescence from the fluorophores of the dissociated biological components 12, here the dissociated cells, is equivalent to reading the unique signature of the biological components. Therefore, the measurement allows obtaining the information of the spatial location of the cells before their dissociation. The method then comprises mapping the optical signatures measured in the dissociated biological components 12 to spatial locations of the cells in the non-dissociated sample 10.

The set of active fluorophore concentrations is a direct proxy for the spatial position of the biological component in the biological sample. Hence, single cell omics data and spatial data are generated from the exact same biological components.

The skilled person will understand that the present invention does not suffer the drawbacks of the known 3D imaging methods with slide-based approaches, such as the issues of aligning consecutive ones of the slides, loss of spatial resolution and information in cutting edges, non-equal resolution along the different axes, labour time, and cost.

The optical encoding will now be explained in detail with reference to Fig.3.

The biological sample 10 is first prepared. This preparation includes fixing and permeabilizing the cells (as an example of biological components) of the biological sample 10 with a fixing agent and a permeabilization agent at step S110.

Suitable fixing agents include but are not limited to Paraformaldehyde/formalin, Methanol, Acetone, DSP and its derivatives, and flash freezing.

The sample 10 can be permeabilized with permeabalization agents. Suitable permeabalization agents include but are not limited to organic solvents and detergents in general such as triton X-100, tween-12, CHAPS, Sarkosyl, DMSO, SDS and Saponin. Some of these agents are capable of both fixing and permeabilizing the sample 10 simultaneously.

Next, the biological sample 10 is stained with the plurality of fluorophores 31, 32, 33, 34 at step S120. The staining is carried out by at least three different fluorophores 31, 32, 33. The three different fluorophores 31, 32, 33 have different excitation spectra and different emission spectra.

In the detailed example, three different fluorophores are used, to create a coordinate system with three dimensions. However, this is not limiting the invention, and other coordinate systems could be contemplated.

The different fluorophores have a first plurality of first fluorophores 31 with a first excitation spectrum and first emission spectrum, a second plurality of second fluorophores 32 with a second excitation spectrum and second emission spectrum, and a third plurality of third fluorophores 33 with a third excitation spectrum and third emission spectrum.

Fourth fluorophores 34 are also used as control fluorophores. The four different fluorophore concentrations are stochiometric, i.e., the concentrations of the fluorophores proportional to each other. It is therefore possible to normalize the encoding fluorophore concentrations 31, 32, 33, by the fourth (control) fluorophore 34. The use of the control fluorophore or control channel relaxes the requirement of having completely spatially uniform fluorophore concentration. The fourth fluorophore 34 is therefore the staining control which is used to normalize the signals from the other three fluorophores and enables control for the different efficiency in staining in different parts of the sample 10.

The initial fluorophore concentrations of all of the fluorophores 31, 32, 33, 34 should be as uniform as possible throughout the sample 10 in order to obtain well-defined fluorophore concentration gradients.

Suitable fluorophores include, for example, cell marker fluorophores such as those that bind to genomic DNA or RNA, those that bind to cell membranes and those that bind any other component of the cell, those directly covalently attached to oligonucleotides or indirectly attached via processes involving probes. Such probes include but are not limited to padlock probes, snail probes, hybridization chain reaction, or nanoballs generated through rolling circle amplification.

If the staining agent is an oligonucleotide or probe that binds directly or indirectly to genomic DNA these fluorophores (31, 32, 33, 34) can be covalently attached to an oligonucleotide whose sequence is found multiple of times in the genomes of multiple species.

If the staining agent is an oligonucleotide or probe that binds directly or indirectly to RNA these fluorophores (31, 32, 33, 34) can be covalently attached to an oligonucleotide or probe whose sequence can be one that occurs many times in the expressed RNA.

Suitable hybridization mixtures to stain the biological object 10, but are not limited to, components such as DMSO, Triton X-100, Tween-12, Saline Sodium Citrate, Dextran sulphate, Herapin, SDS, Formamide, Denhardt's solution, and EDTA.

If the staining agent is an oligonucleotide, then the biological sample 10 can be heated and cooled one or more times to promote annealing.

In one aspect, it is contemplated to have two oligonucleotides with identical sequence that each have two fluorophores covalently attached.

The fluorophores 31, 32, 33, 34 are located as far apart as possible on the oligonucleotide and/or as spectrally distinct as practically possible to avoid any non-radiative energy transfer.

It should be noted that in the case that the fluorophores 31, 32, 33, 34 are directly or indirectly covalently attached to oligonucleotides, one or more of the fluorophores 31, 32, 33, or 34 can be attached to any given oligonucleotide. In the case that the fluorophores 31, 32, 33, 34 are attached to the same oligonucleotide, optimizing the technique involves maximizing the physical and spectral distance between the fluorophores 31, 32, 33, 34. In the case that the fluorophores 31, 32, 33, 34 are directly or indirectly attached to the oligonucleotides, the sequences of those oligonucleotides should be found in the genome of the species of the biological object 10 being stained.

In the example of the oligonucleotides covalently attached to the fluorophores or probes, they can be annealed to the genomic DNA or RNA of the biological components 12 of the biological sample 10 with a heating and cooling step.

It should be noted that after staining and before altering the fluorophore concentration, the biological sample 10 can be washed to remove unbound stain and post fixing. Suitable washing agents include, but are not limited to, PBS, TBS, Maleic acid buffer and HBSS. Suitable post-fixing agents include, but are not limited to, Paraformaldehyde/formalin, Methanol, Acetone, DSP and its derivatives. Washing the biological sample 10 after staining can improve signal to noise ratio during the further steps of the process.

The next step is to prepare the biological sample 10 for the optical encoding process. This can include mounting the sample 10, possibly dehydration for particular clearing protocols, and chemical clearing of the sample 10. It is often convenient to mount the sample 10 in a hydrogel, which provides protection and mechanical support during processing. Organic-solvent-based clearing protocols such as BABB and the DISCO family, require a dehydration of the sample 10 before clearing by immersion in the organic solvent.

Suitable mounting agents include, but are not limited to, low melting point agarose, agarose, Polyacrylamide and Matrigel.

Suitable dehydration agents include, but are not limited to, methanol, ethanol, propanol, and tetrahydrofuran (THF).

Suitable clearing agents and methods include, but are not limited to, BABB, CLARITY, PACT, PARS, 3DISCO, iDISCO, Spalteholz, Glycerol and CUBIC. For simplicity, in the remainder of this detailed description of the invention we describe the clearing as being done using the BABB protocol, although other clearing protocols could be used.

The next step in the process is imaging and altering at step S130 the active concentrations of the fluorophores 31, 32, 33 34, thereby creating the plurality of gradients of active fluorophore concentrations in the biological sample 10. This step S130 can be done using an optical encoding system used to alter and/or image the distribution of active fluorophores 50.

There is a plurality of mechanisms that can be used to optically alter the active fluorophore concentrations. Depending on the choice of fluorophores used 31,32,33,34 these mechanisms include, but are not limited to, photo-bleaching, photo-switching, or photo-activation. For simplicity, in this detailed description of the invention we describe the use of photo-bleaching.

One method to perform the optical encoding S100 is to use an optical instrument that employs the scanning of spatially non-uniform illumination. The desired active fluorophore distribution in the sample 10 results from the specific non-uniform illumination and scanning patterns employed. Examples of such instruments include, but are not limited to, a light sheet microscope, a confocal microscope, or a 2-photon microscope. For simplicity, in the remainder of this detailed description of the invention we describe the imaging and altering at step S130 (i.e. the optical encoding) as being done using a SPIM microscope.

An alternative to the use of scanning-type instruments for optical encoding is to use illumination with a collimated beam that has the appropriate intensity profile. For example, a beam with a logarithmic intensity profile can be used to bleach a linear gradient along one dimension of a uniformly stained sample without the need for scanning.

The plurality of gradients is created by altering S130 active fluorophore concentrations of ones of the plurality of fluorophores 31, 32, 33, 34 in the biological sample 10 after the staining S120.

Optimally, the plurality of gradients of the active fluorophore concentrations are orthogonal to each other. A first gradient is a gradient of first fluorophores with a first excitation spectrum and first emission spectrum, a second gradient is a gradient of the second fluorophores with a second excitation spectrum and second emission spectrum, and a third gradient is the gradient of third fluorophores with a third excitation spectrum and third emission spectrum.

In other words, each cell can be identified by a unique optical signature represented by the three fluorophores, with one fluorophore per dimension.

It will be appreciated that a minimum of three fluorophores is required to unambiguously encode each of the three spatial dimensions. The optical signature is a set of sample coordinates represented by the three fluorophores, with one fluorophore per dimension. A coordinate system with further spatial dimensions can be contemplated.

A calibration sample is first imaged in 3-dimensions in all 4 channels, and successful staining confirmed.

Altering the concentration of active fluorophores 31, 32, 33, 34 can then be done via photo-activation, photo-switching, or photo-bleaching, depending on the types of the fluorophores 31, 32, 33, 34 used. The resulting concentration distributions of the active fluorophores 31, 32, 33, 34 could include any that provide non-ambiguous positional information to one or more cells within the biological sample 10.

In one aspect, a Selective Plane Illumination Microscope (SPIM) or other light sheet microscope 50 is used to image the cells. The calibration sample is bleached in a single plane, i.e. a different non-overlapping plane in a distinct part of the calibration sample for each channel, or each of the channels to be bleached.

The reduction in the concentration of the active fluorophores 31, 32, 33, 34 is recorded by imaging the biological sample 10 as the sample is bleached over time. For each of the fluorophores 31, 32, 33, 34, bleaching calibration graphs 48 are generated that determine the bleaching parameters required to bleach to the desired level of the active fluorophore concentration. The bleaching parameters for each of the three channels are read off the calibration graphs generated from bleaching each of the fluorophores 31, 32, 33, 34 in the calibration sample. The bleaching calibration graphs 48 can be used to determine the exposure for each position along the axis to achieve a desired gradient in the biological sample 10.

The light sheet microscope 50 can quickly bleach a given plane in the biological sample 10 while leaving the remainder relatively unaffected. In the case that a light sheet microscope is used, the light sheets can be generated via a cylindrical lens or by a rapidly translated laser line or lines such as via a galvo mirror or resonant scanner. In addition, a light sheet microscope can easily create 3D images of the resulting bleached patterns, which can be used to confirm the obtained active fluorophore distribution.

Post bleaching imaging of the biological sample 10 can be used when mapping the fluorescence measured from the dissociated biological components 12, to the spatial location of the biological components 12 in the non-dissociated sample, as explained later in the disclosure. Post bleaching imaging of the biological sample 10 can be used for the mapping along with the single colour controls that determine the colour space mapping between the imaging/altering system and dissociated cell detection.

As mentioned before, it is desirable to create three orthogonal gradients, i.e. orthogonal monotonic distributions for example. In practice, linear distributions tend to optimize the signal to noise ratios and make the positional accuracy spatially uniform. After the desired patterns are written into the fluorophores, the patterns are imaged in 3D to quantitatively record the actual patterns achieved.

Before altering the intensities of the fluorophores, the Pearson correlation coefficient should be strong for every altered fluorophore 31, 32, 33 with respect to the control fluorophore 34 being used, where each point in the correlation analysis is the intensity of the fluorophores of a single cell. For example, the Pearson correlation coefficient is preferably more than 0,2, more preferably more than 0,5, and even more preferably at least 0,9.

One technique for carrying out the step of altering S130 of the active fluorophore concentrations is photobleaching using a light sheet 40 with differential periods of exposure of sections of the biological sample (10) to the light sheet (40).

The bleaching parameters for each of the 3 channels are derived from the calibration graphs generated from bleaching each fluorophore in the calibration sample.

One approach consists in generating a linear active fluorophore distribution that should result in homogeneous resolution over the axis. The produced gradient is linear rather than an exponential decay, so that the positional error at any position along the axis is uniform. If a gradient with an exponential decay is produced, e.g., by linearly changing the exposure along the axis, then different amounts of positional error along the axis are obtained depending on the gradient of the curve.

The first-dimension x is bleached using a light sheet perpendicular to the x dimension that excites one of the fluorophores 31 that is parked for different amounts of time in the biological sample 10.

The longer the light sheet is placed in each position, the lower the resulting concentration of unbleached fluorophores in the corresponding plane. Alternatively, different regions of the biological sample 10 can be scanned a different number of times to bleach a gradient, or the laser power could be spatially varied. A multiple scanning is effectively the same as just changing the exposure.

The second dimension y is bleached by rotating the sample by 90 degrees about the vertical axis (z) and then repeating the process used for the x-dimension bleaching with the laser line appropriate for the second fluorophore 32.

The third-dimension z is bleached using a finite-height light sheet with the laser line appropriate for the third fluorophore 33 that is scanned over the biological sample 10 for equal amounts of time. Between scans the sample 10 is incrementally raised (translated along the z-axis) out of the path of the light sheet, so that different regions in the sample are illuminated different numbers of times, and a gradient of active fluorophores 33 is bleached along the third dimension.

As an alternative to the process described in the previous paragraph, to bleach the z-dimension the sample 10 could be rotated by 90 degrees around a horizontal axis and then repeating the process used for the y-dimension bleaching with the laser line appropriate for the third fluorophore 33.

An alternative to the steps described in the previous paragraphs, which assume successive bleaching of the sample 10 along the 3 axes using a SPIM instrument with illumination along a single axis, is to constrict an instrument with the possibility of illumination along two or more axis. This l allows bleaching along multiple axes, either sequentially or simultaneously, without the need to rotate the sample between bleachings.

The result is a biological sample 10 with orthogonal active fluorophore gradients along the three spatial dimensions, plus the unbleached control fluorophore 34.

Post-bleaching, the concentration profiles of the 4 fluorophores are determined by imaging the biological sample 10 in 3-dimensions in all 4 channels.

This is not the only method and the altering can be carried out by one of photoactivation or photoswitching.

The next step is "unclear" the sample and return it to its pre-cleared state. This can be done by reversing the clearing protocol and possibly rehydrating for those protocols that required dehydrating. Suitable unclearing agents include, but are not limited to, Methanol, Ethanol and propanol. Suitable rehydration agents include, but are not limited to, PBS, TBS, Maleic acid buffer and HBSS.

The next step comprises disassociating S200 the biological components 12 from the biological sample 10 to create individual biological components 12 of the biological components 12.

The sample 10 can be dissociated enzymatically using enzymes including, but not limited to, Collagenase, Dispase and Trypsin. Tissue can also be dissociated mechanically by vortexing, sonication, a manual or motorized pestel, or use of a specialized tissue dissociator such as the gentleMACS. Combinations of enzymatic and mechanical methods can also be utilized to dissociate the biological sample 10.

The next step involves separating the biological components 12 into separate compartments for downstream processing. The biological components can be separated by means of a FACS machine, via a microfluidics device, or dilution. Compartments include wells of multiwell plates, fabricated microwells, tubes, spots on slides, and bubbles of water-in-oil emulsions.

After dissociation, the next step is optical decoding S300 which comprises measuring the fluorescence S310 of the dissociated biological components and mapping S320 those fluorescence measurements to the spatial location of the biological components in the non-dissociated sample.

This is now explained in the following with reference to figure 4.

First, the fluorescence from the dissociated biological components 12 is read. More precisely, either during or after the components separation, the fluorescent intensities of the plurality of fluorophores should be read in order to determine where in the sample 10 that biological component 12 originated.

To read the intensity of the fluorophores 31, 32, 33, 34, a measuring system (80) can be used comprising an excitation system 60 such as a laser, LED, or fluorescent lamp to excite the fluorophores and a detector system 70 such as a camera, PMT or other type of photodetector of an optical decoding system 300 to measure the fluorescence. This detection (step S310) can be made within the context of a Fluorescence-activated cell sorting (FACS) machine, a fluorescent plate or slide reader, or a fluorescence excitation/detection setup incorporated within a microfluidics device for example.

Any omics can then be performed on the biological components 12 in the individual compartments and the location of each component 12 in the original biological sample 10 derived from the levels of fluorescence of the 3 axes normalized to the control channel.

The measured levels of fluorescence are mapped at step S320 to the spatial locations of the non-dissociated cells in the biological sample 10. Each colour combination measured after the dissociation is thereby assigned to a unique location in the physical space in the biological sample 10. Indeed, the set of active fluorophore concentrations is a direct proxy for spatial position of the biological components in the biological sample.

For each biological component 12, the levels recorded in the three bleaching channels F1, F2, F3 can be normalized by the control non-bleached channel F4. The normalization means dividing the levels of the bleaching channels F1, F2, F3 by the level to the control channel F4.

Typically, linear distributions of the active fluorophores can be used so that the resulting optical signatures are direct proxies for the original locations of the biological components 12 within the biological sample (10). The mapping between the normalized "colour code" of the cell, (F1/F4, F2/F4, F3/F4) and the cells original position x,y,z in the biological sample 10 is then a simple set of linear equations.

In other words, the original 3D positions of the biological components 12 are reconstructed by normalizing the levels of the active fluorophores of each altered bleached, photo activated or switched channel by that of the control unaltered channel to produce an optical signature for each component 12. The resulting optical signature serves as a proxy for the location in physical space, i.e. the original position {x,y,z} of each component 12 in the biological sample 10. This approach can be done if the distributions of the active fluorophore concentrations were in the optical encoding form a basis, in linear algebraic terms.

The optical encoding has imposed a coordinate system on the biological sample and the concentrations of active fluorophores are, for the biological component, the set of spatial coordinates in said coordinate system.

Before disassociation, it is possible to measure the colour code distribution or values, (I1, I2, I3, & I4), in the light sheet microscope 50 that was used for bleaching. As mentioned above, for orthogonal, linear distributions of the active fluorophores, this mapping from "colour-space" to "physical-space" is a linear one.

After dissociation, it is possible to record the fluorescent levels (F1,F2,F3, &F4) in the dissociated cells, for example measured by the detector system 70, e.g. the FACS machine.

Ideally the response of the system used to image the biological sample 10 before dissociation but after altering the distribution of active fluorophores (e.g. a light sheet microscope 50), and the response of the detector system 70 used to detect the fluorescent levels in the dissociated cells (e.g. a FACS machine) to a given concentration of active fluorophores would be identical.

The skilled person will understand that even when trying to match the responses measured on the optical encoding system used to alter and/or image the distribution of active fluorophores and the system used to readout the fluorescence once the biological components 12 have been dissociated as closely as possible, the responses will not be identical. For example, if the optical encoding system used to alter and/or image the distribution of active fluorophores is a light sheet microscope and the system used to readout the fluorescence once the biological components 12 have been dissociated a detector system 70, similar laser lines for excitation can be used in the light sheet microscope 50 and in the detector system 70 to match the responses as closely as possible. However, the responses will not be identical. This is due for example to slightly different lasers, completely different detectors, different filters and sample media.

It is therefore proposed to have a mapping from the recorded fluorescence after dissociation colour-space, {F1/F4, F2/F4, F3/F4}, into the recorded fluorescence before dissociation colour-space, {I1/I4 ,I2/I4, I3/I4}, which can then be mapped into physical-space, {x,y,z}, as described above.

The mapping of measured colour space of dissociated cells to physical space, i.e. the mapping {F1, F2, F3,F4} → {x,y,z}, therefore first involves mapping measured colour space of dissociated cells to the imaged colour space in the imaging system (50) used to image the sample (10), i.e. the mapping {F1, F2, F3, F4} → {I1, I2, I3, I4}. The mapping from imaged colour space to physical space, {I1/I4, I2/I4, I3/I4} → {x,y,z}, is straightforward because we have the voxel data set from the post-bleached sample scan: in this voxel data, the voxel *coordinates* represent physical space, {x,y,z}, and the voxel *values* represent the imaged colour space, {I1/I4 ,I2/I4, I3/I4}. This mapping from the measured colour-space of dissociated cells to physical space is done by calibrating the responses of the system used to alter and/or image the distribution of active fluorophores 50 and the system used to readout the fluorescence once the biological components 12 have been dissociated 80 using four calibration samples that each contain only one of the four fluorophores 31, 32, 33, 34. A mapping between the imaging system 50 and the detector system 80 response curves then gives the desired relationship between the recorded fluorescence after dissociation colour-space, {F1, F2, F3, F4} and the recorded fluorescence before dissociation colour-space, {I1 ,I2, I3, I4}, and therefore to the physical-space coordinates {x,y,z}, i.e. {F1, F2, F3, F4} → {I1 ,I2, I3, I4}→ {I1/I4, I2/I4, 13/14} → {x,y,z}. Suitable methods for this mapping include, but are not limited to, a histogram matching algorithm for each channel, or the use of artificial intelligence methods.

The histogram matching is done using the fluorescent signals measured in all four channels for four biological samples each stained with only a single type of fluorophore, for both the system used to alter and/or image the distribution of active fluorophores and the system used to readout the fluorescence once the biological components 12 have been dissociated. The measured optical signature of each biological component 12 after dissociation is then mapped to the corresponding optical signature in the system used image the 3D fluorophore distribution before dissociation.

From this resulting adapted optical signature, the pre-dissociation physical location of the biological component 12 can be read out by comparing the optical signature to the fluorophore concentrations at every physical location within the 3D image (post altering the distribution of active fluorophores) using a distance metric such as the Euclidean distance and choosing the most probable location where this metric is smallest. Other suitable distance metrics for this mapping include, but are not limited to, Pearson, Kendall's tau, Manhattan and Spearman rank correlation distances.

It is possible that there will be cases in which the optical signatures cannot be encoded as monotonic gradients, but the optical signatures can still be used in some other way to produce unambiguous location information. e.g. if more than four suitable fluorophores were available, two of the fluorophores could be combined along one axis to render positioning unambiguous even though either fluorophore taken alone could not provide unambiguous information. Then a mapping step can instead be used that maps the resulting biological components 12 to the most likely location of their origin in the sample 10 by comparing the ensemble of normalized optical signatures of the biological components 12 to the optical signatures encoded in the sample 10 (which are read out at the post bleaching imaging stage) using any form of suitable distance metric such as the Euclidean distance. Other suitable distance metrics for this mapping include, but are not limited to, Pearson, Kendall's tau, Manhattan and Spearman rank correlation distances.

If sufficient biological components 12 are mapped to their expected location of origin in the biological sample 10 it is possible to reconstruct the original tissue (S400).

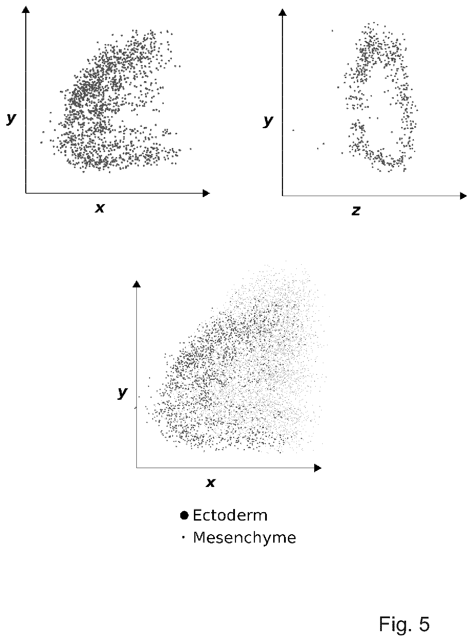

Fig. 5 gives an example of data obtained with the method and system of the present disclosure, with the protocols detailed below.

### Imaging and bleaching

Samples were imaged and bleached on an inhouse-built cylindrical lens based OPTiSPIM light sheet mesoscope1. Briefly, a 50mW 405nm, 50mW 488nm, and 50mW 639nm lasers were used to image and bleach the Alexa405, FAM and Alexa647 fluorophores respectively. A 5mW 543nm laser was used to image the internal staining control TAMRA fluorophore. 447BP60, 525BP50, 585BP60, and 700BP75 band pass filters were used for detecting the Alexa405, FAM, TAMRA and Alexa647 fluorophores respectively. A 2.5×N Plan air objective (Leica, NPLAN, NA=0.07) was used to illuminate the samples. A 5×N Plan Epi air objective (Leica, NPLAN EPI, NA=0.12, WD=14 mm) and a 12 bit cooled Hamamatsu ORCA-ER C4742-80 CCD camera was used for detection.

A calibration sample was used to calibrate the bleaching, by placing light sheets of each laser line in a fixed position and monitoring the reduction in intensity of fluorescent signal with time of exposure. Bleaching exposure times for different positions of the light sheet in test samples are determined through reading off of these calibration graphs (one for each fluorophore/laser line).

### Processing of bleached samples

After bleaching, the sample 10 received one quick wash and two longer washes (>4hours) in Methanol. The sample 10 was then removed from the LMP agarose and rehydrated with one quick wash and two longer washes (>4 hours) in PBS. For the experiments where the limb ectoderm and mesenchyme are analyzed separately the ectoderm was removed from the limb with tweezers after incubating the limb bud in 0.5% Trypsin-EDTA 10x (without phenol red - Gibco 15400-054) for 15 minutes at room temperature.. For any other experiment the tissues were digested with 0.22um filtered 10mg/ml Collagenase/dispase in PBS (with 1/1000000 triton) at 37C 600 RPM for 2 hours. Biological samples 10 were periodically mechanically agitated with a gilson pipette. Following tissue dissociation, the sample 10 was centrifuged at 600g for 5minutes at room temperature. Cell pellets were resuspended in 0.5-1ml PBS and strained through a 40µm strainer and processed on a FACS analyzer (Fortessa). It should be noted that this is not a limiting example, and other devices could be used, such as a cell sorter (FACSAria).

### Detector system after dissociation

In the example shown, cells were simply analyzed for their fluorescent levels, with a Fortessa FACS analyzer.. A FACS gate for Side scatter area and Forward scatter was used to remove debris (gate P1). Singlets were then selected with a Forward scatter height versus area gate (gate P2). A PE-A (Corresponding to a 586BP15 band-pass filter to detect the TAMRA staining control) versus Forward scatter gates was used to select the fluorescent cells and remove any remaining debris (gate P3).

PACB (Corresponding to a 450BP50 band-pass filter to detect Alexa405), FITC-A (Corresponding to a 530BP28 band-pass filter to detect FAM) and APC-A (Corresponding to a 670BP14 band-pass filter to detect Alexa647) fluorescent channel levels were normalized to the PE-A (to detect TAMRA) fluorescent channel level to generate the data shown in figure 5. Thus for simplicity, in this example the reconstruction assumes the responses of the FACS machine and the SPIM machine are identical and colour space was assumed to be a direct proxy for physical space (as described in paragraphs 104-107) since linear gradients were generated in all dimensions.

Figure 5 shows the result of a reconstruction of a E10.5 hind limb bud from a mouse embryo that has undergone the C3PO process in all 3 dimensions. The ectoderm cells pre-dissociation are known to form a cap over the mesenchyme with a concavity in the center and a thickened curved edge corresponding to the well characterized apical ectodermal ridge (AER). The top left panel of figure 5 show that indeed the ectoderm cells are found with a curved thickening around exactly where the AER is expected to be found (low x-dimension). The top right panel shows a transversal slab of the ectoderm cells with a clear concavity in the center of the ring of ectoderm cells as would be expected. There is also very little ectoderm cells detected on the straight edge side of the limb bud (high x-dimension). This was where the limb bud was cut off from the rest of the embryo so there should be no ectoderm along this edge exactly as can be seen in the reconstruction. Finally in the bottom panel we show the limb ectoderm cells (large dots) with the limb mesenchymal cells (small dots). The ectoderm cells should surround the mesenchyme since this layer sits directly on the mesenchyme in the wildtype limb bud, which is exactly the configuration that we observe in the reconstruction. Overall, therefore the reconstructed configuration of the limb bud is very similar to the configuration pre-dissociated demonstrating that the method described in the present disclosure is functional.

### Reference number list

1 system
10 biological sample. Examples include, but are not limited to, organs, embryos, or biopsies.
12 biological component. Examples include, but are not limited to, cells or clusters of cells.
31, 32, 33 fluorophores
34 control fluorophores
40 Light Sheet
48 calibration graphs
50 System used to alter and/or image the distribution of active fluorophores
60 excitation system
70 detection system
80 Optical measuring system used to readout the fluorescence once the cell have been dissociated
90 Mapping unit
100 optical encoding unit
300 optical decoding system
{F1,F2,F3,F4} recorded fluorescence using (70) after dissociation colour-space,
{I1,I2,I3,I4} recorded fluorescence using (50) before dissociation colour-space
{x,y,z} physical-space

## Claims

1. A method for determining spatial location of one or more biological components (12) in a biological sample (10), the method comprising:
- optically encoding (S100) the biological components (12) in the biological sample (10) with a plurality of fluorophores (31, 32, 33, 34) to create a combination of active fluorophore concentrations within the biological sample (10), indicative of the spatial location of the one or more biological components (12), wherein the step of optical encoding the biological components, comprises optically encoding a coordinate system on the biological sample, and the combination of active fluorophore concentrations is, for the biological components, a set of coordinates in said coordinate system;
- dissociating (S200) the biological components (12) from the biological sample (10) to create dissociated biological components (12) of the biological components (12); and
- optically decoding (S300) by measuring the fluorescence (S310) of the dissociated biological components (12) and mapping (S320) to the spatial location of the biological components (12) in the non-dissociated sample (10), wherein the step of optically decoding comprises determining, for each dissociated biological component, the set of coordinates in said sample coordinate system, to obtain the spatial location of each of the biological components in the non-dissociated sample (10).

2. The method of claim 1, wherein the coordinate system on the biological sample is an orthogonal cartesian coordinate system.

3. The method of any of claims 1 to 2, wherein creating the spatial combination of active fluorophore concentrations comprises staining (S120) the biological sample (10) with said plurality of fluorophores (31, 32, 33, 34).

4. The method of any of claims 1 to 3, wherein multiple different fluorophores are attached to a single molecule, for example covalently attached to an oligonucleotide.

5. The method of claim 4, wherein multiple different fluorophores attach to parts of the biological component with constant ratios.

6. The method of any of claims 3 to 5, further comprising altering (S130) active fluorophore concentrations of ones of the plurality of fluorophores (31, 32, 33, 34) in the biological sample (10) after the staining (S120).

7. The method according to any of claims 1 to 6, comprising creating a plurality of gradients of active fluorophore concentrations in the biological sample (10), in particular the plurality of gradients of active fluorophore concentrations being orthogonal to each other.

8. The method according to claim 6 or 7, wherein the altering (S130) of the active fluorophore concentrations is carried out by one of photobleaching, photoactivation or photo-switching, in particular wherein the altering (S212) is carried out by photobleaching using a light sheet (40) with differential periods of exposure of regions of the biological sample (10) to the light sheet (40).

9. The method according to claim 8, comprising measuring fluorescence levels of a calibration sample before and after the alteration of the active fluorophore concentrations, to generate bleaching calibration graphs (48) that define the bleaching parameters to use during altering the resulting level of the active fluorophore concentration in the sample of interest (10).

10. The method according to any of the above claims, wherein the optical encoding (S100) is carried out by different fluorophores (31, 32, 33), which have different excitation spectra and different emission spectra defining at least three fluorescence channels.

11. The method according to any of the above claims, comprising using additional fluorophores (34) as control fluorophores, in particular the method further comprising normalising the measured fluorescence levels of the non-control fluorophores (31, 32, 33) with respect to the fluorescence levels of the control fluorophores (34).

12. The method according to any of the above claims, comprising mapping measured fluorescence levels of the different fluorophores (31, 32, 33) of the dissociated biological components (12) to the physical space in the biological sample (10) before dissociation, such that each colour combination measured after the dissociating is assigned to probable locations in physical space.

13. The method according to claim 11, where the mapping of measured levels of fluorescence of dissociated biological components (12) to the physical space comprises imaging the biological sample (10) before dissociation and mapping measured colour space of the dissociated biological components to the measured colour space in the system used to image the biological sample (10) before dissociation, for example by means of a histogram matching algorithm for each fluorescence channel.

14. The method according to claim 12 or 13, where single fluorophore stained control samples are used to determine cross-talk between pair-wise combinations of channels, in both the system used to image the biological sample (10) before dissociation and the system used to measure the signals of the biological components (12) after dissociation, to correct channel data in both systems before histogram matching each channel between the two instruments.

15. The method of any of the above claims, further comprising reconstructing of the biological sample (10) from the measured fluorescence.

16. System for determining spatial location of one or more biological components (12) in a biological sample (10), comprising:
- an optical encoding unit (50) provided for optically encoding the biological components (12) in the biological sample (10) with a plurality of fluorophores (31, 32, 33, 34) to create a combination of active fluorophore concentration indicative of the spatial location of the one or more biological components (12), wherein the optical encoding unit is adapted to optically encode a sample coordinate system on the biological sample, with the combination of active fluorophore concentrations being, for the biological components, a set of coordinates in said sample coordinate system;
- an optical measuring system (80) to measure fluorescence after dissociation, comprising one of a fluorescent activated cell sorter or analyser, a microfluidic device
- a mapping unit (90), configured to mapping the fluorescence measured from the dissociated biological components (12), to the spatial location of the biological components (12) in the non-dissociated sample (10), wherein the mapping unit (90) is adapted to determine, for each dissociated biological component, the set of coordinates in said sample coordinate system, to obtain the spatial location of each of the biological components in the non-dissociated sample (10)..

17. The system according to claim 16, wherein the optical encoding unit comprises an imaging unit to alter the spatial distribution of plurality of fluorophore concentrations and measure the spatial distribution of the plurality of fluorophore concentrations after alteration, in particular wherein the imaging unit comprises a Selective Plane Illumination Microscope or other Light Sheet Microscope (50) for imaging the biological sample (10) before dissociation.

18. The system according to any of claims 16 or 17, wherein the sample coordinate system is an orthogonal cartesian coordinate system.

## Patentansprüche

1. Verfahren zum Bestimmen der räumlichen Lage einer oder mehrerer biologischer Komponenten (12) in einer biologischen Probe (10), das Verfahren umfassend:
- optisches Codieren (S100) der biologischen Komponenten (12) in der biologischen Probe (10) mit einer Vielzahl von Fluorophoren (31, 32, 33, 34), um eine Kombination von aktiven die räumliche Lage der einen oder mehreren biologischen Komponenten (12) anzeigenden Fluorophorkonzentrationen innerhalb der biologischen Probe (10) zu erzeugen, der Schritt des optischen Codierens der biologischen Komponenten bei welchem das optische Codieren eines Koordinatensystems auf der biologischen Probe umfasst, und die Kombination von aktiven Fluorophorkonzentrationen für die biologischen Komponenten ein Koordinatensatz in dem Koordinatensystem ist;
- Dissoziieren (S200) der biologischen Komponenten (12) von der biologischen Probe (10), um von den biologischen Komponenten (12) dissoziierte biologische Komponenten (12) zu erzeugen; und
- optisches Decodieren (S300) durch Messen der Fluoreszenz (S310) der dissoziierten biologischen Komponenten (12) und Abbilden (S320) auf die räumliche Lage der biologischen Komponenten (12) in der nicht dissoziierten Probe (10), der Schritt des optischen Decodierens umfassend das Bestimmen des Koordinatensatzes in dem Probenkoordinatensystem für jede dissoziierte biologische Komponente, um die räumliche Lage jeder der biologischen Komponenten in der nicht dissoziierten Probe (10) zu erhalten.

2. Verfahren nach Anspruch 1, bei welchem das Koordinatensystem auf der biologischen Probe ein orthogonales kartesisches Koordinatensystem ist.

3. Verfahren nach einem der Ansprüche 1 bis 2, das Erzeugen der räumlichen Kombination von aktiven Fluorophorkonzentrationen umfassend das Färben (S120) der biologischen Probe (10) mit der Vielzahl von Fluorophoren (31, 32, 33, 34).

4. Verfahren nach einem der Ansprüche 1 bis 3, bei welchem mehrere verschiedene Fluorophore an ein einzelnes Molekül gebunden sind, zum Beispiel kovalent an ein Oligonukleotid gebunden sind.

5. Verfahren nach Anspruch 4, bei welchem mehrere verschiedene Fluorophore an Teile der biologischen Komponente mit konstanten Verhältnissen gebunden sind.

6. Verfahren nach einem der Ansprüche 3 bis 5, ferner umfassend ein Ändern (S130) der aktiven Fluorophorkonzentrationen von einem der Vielzahl von Fluorophoren (31, 32, 33, 34) in der biologischen Probe (10) nach dem Färben (S120).

7. Verfahren nach einem der Ansprüche 1 bis 6, umfassend das Erzeugen einer Vielzahl von Gradienten von aktiven Fluorophorkonzentrationen in der biologischen Probe (10), bei welcher insbesondere die Vielzahl von Gradienten von aktiven Fluorophorkonzentrationen orthogonal zueinander ist.

8. Verfahren nach Anspruch 6 oder 7, bei welchem das Ändern (S130) der aktiven Fluorophorkonzentrationen durch eines von Photobleichen, Photoaktivierung oder Photoumschaltung durchgeführt wird, bei welchem insbesondere das Ändern (S212) durch Photobleichen unter Verwendung einer Lichtplatte (40) mit unterschiedlichen Belichtungsperioden von Bereichen der biologischen Probe (10) mit der Lichtplatte (40) durchgeführt wird.

9. Verfahren nach Anspruch 8, umfassend das Messen von Fluoreszenzniveaus einer Kalibrierungsprobe vor und nach der Änderung der aktiven Fluorophorkonzentrationen, um Bleichparameter definierende Bleichkalibrierungsgraphen (48) zu erzeugen, die während der Änderung des resultierenden Niveaus der aktiven Fluorophorkonzentration in der Probe von Interesse (10) zu verwenden sind.

10. Verfahren nach einem der vorstehenden Ansprüche, bei welchem die optische Codierung (S100) durch verschiedene Fluorophore (31, 32, 33) durchgeführt wird, die verschiedene Anregungsspektren und verschiedene Emissionsspektren aufweisen, die mindestens drei Fluoreszenzkanäle definieren.

11. Verfahren nach einem der vorstehenden Ansprüche, umfassend das Verwenden zusätzlicher Fluorophore (34) als Kontrollfluorophore, das Verfahren insbesondere ferner umfassend das Normalisieren der gemessenen Fluoreszenzniveaus von Nicht-Kontrollfluorophoren (31, 32, 33) in Bezug auf die Fluoreszenzniveaus der Kontrollfluorophore (34).

12. Verfahren nach einem der vorstehenden Ansprüche, umfassend das Abbilden gemessener Fluoreszenzniveaus der verschiedenen Fluorophore (31, 32, 33) der dissoziierten biologischen Komponenten (12) auf den physikalischen Raum in der biologischen Probe (10) vor Dissoziation, so dass jede nach der Dissoziation gemessene Farbkombination wahrscheinlichen Stellen im physikalischen Raum zugewiesen wird.

13. Verfahren nach Anspruch 11, das Abbilden gemessener Fluoreszenzniveaus von dissoziierten biologischen Komponenten (12) auf den physikalischen Raum umfassend das Abbilden der biologischen Probe (10) vor Dissoziation und das Abbilden gemessener Farbräume der dissoziierten biologischen Komponenten auf den gemessenen Farbraum in dem System, das verwendet wird, um die biologische Probe (10) vor Dissoziation abzubilden, beispielsweise mittels eines Histogrammanpassungsalgorithmus für jeden Fluoreszenzkanal.

14. Verfahren nach Anspruch 12 oder 13, bei welchem mit einzelnen Fluorophoren gefärbte Kontrollproben verwendet werden, um das Übersprechen zwischen paarweisen Kombinationen von Kanälen sowohl in dem System, das verwendet wird, um die biologische Probe (10) vor Dissoziation abzubilden, als auch in dem System, das verwendet wird, um die Signale der biologischen Komponenten (12) nach Dissoziation zu messen, zu bestimmen, um Kanaldaten in beiden Systemen vor der Histogrammanpassung jedes Kanals zwischen den zwei Instrumenten zu korrigieren.

15. Verfahren nach einem der vorstehenden Ansprüche, ferner umfassend das Rekonstruieren der biologischen Probe (10) aus der gemessenen Fluoreszenz.

16. System zum Bestimmen der räumlichen Lage einer oder mehrerer biologischer Komponenten (12) in einer biologischen Probe (10), umfassend:
- eine optische Codiereinheit (50), die zum optischen Codieren der biologischen Komponenten (12) in der biologischen Probe (10) mit einer Vielzahl von Fluorophoren (31, 32, 33, 34) bereitgestellt ist, um eine Kombination von aktiven die räumliche Lage der einen oder mehreren biologischen Komponenten (12) anzeigenden Fluorophorkonzentrationen zu erzeugen, wobei die optische Codiereinheit ausgelegt ist, um ein Probenkoordinatensystem auf der biologischen Probe optisch zu codieren, wobei die Kombination von aktiven Fluorophorkonzentrationen für die biologischen Komponenten ein Koordinatensatz in dem Probenkoordinatensystem ist;
- ein optisches Messsystem (80) zum Messen von Fluoreszenz nach Dissoziation, umfassend eines von einem fluoreszenzaktivierten Zellsortierer oder -analysator, einer mikrofluidischen Vorrichtung
- eine Abbildungseinheit (90), die eingerichtet ist, um die von den dissoziierten biologischen Komponenten (12) gemessene Fluoreszenz auf die räumliche Lage der biologischen Komponenten (12) in der nicht dissoziierten Probe (10) abzubilden, wobei die Abbildungseinheit (90) angepasst ist, um für jede dissoziierte biologische Komponente den Koordinatensatz in dem Probenkoordinatensystem zu bestimmen, um die räumliche Lage jeder der biologischen Komponenten in der nicht dissoziierten Probe (10) zu erhalten.

17. System nach Anspruch 16, die optische Codiereinheit umfassend eine Abbildungseinheit, um die räumliche Verteilung der Vielzahl von Fluorophorkonzentrationen zu ändern und die räumliche Verteilung der Vielzahl von Fluorophorkonzentrationen nach der Änderung zu messen, insbesondere umfasst die Abbildungseinheit ein selektives Ebenenbeleuchtungsmikroskop oder ein anderes Lichtplattenmikroskop (50) zum Abbilden der biologischen Probe (10) vor Dissoziation.

18. System nach einem der Ansprüche 16 oder 17, bei welchem das Probenkoordinatensystem ein orthogonales kartesisches Koordinatensystem ist.

## Revendications

1. Procédé de détermination d'un emplacement spatial d'un ou de plusieurs composants biologiques (12) dans un échantillon biologique (10), le procédé comprenant :
- encoder de manière optique (S100) des composants biologiques (12) dans l'échantillon biologique (10) avec une pluralité de fluorophores (31, 32, 33, 34) pour créer une combinaison de concentrations de fluorophores actifs à l'intérieur de l'échantillon biologique (10), indicative de l'emplacement spatial des un ou plusieurs composants biologiques (12), dans lequel l'étape consistant à encoder de manière optique des composants biologiques comprend un encodage optique d'un système de coordonnées sur l'échantillon biologique, et la combinaison de concentrations de fluorophores actifs est, pour les composants biologiques, un ensemble de coordonnées dans ledit système de coordonnées ;
- dissocier (S200) les composants biologiques (12) de l'échantillon biologique (10) pour créer des composants biologiques dissociés (12) desdits composants biologiques (12) ; et
- décoder de manière optique (S300) par une mesure de la fluorescence (S310) desdits composants biologiques dissociés (12) et une mise en correspondance (S320) avec l'emplacement spatial desdits composants biologiques (12) dans l'échantillon non dissocié (10), dans lequel l'étape de décodage optique comprend la détermination, pour chaque composant biologique dissocié, de l'ensemble de coordonnées dans ledit système de coordonnées d'échantillon, pour obtenir l'emplacement spatial de chacun desdits composants biologiques dans l'échantillon non dissocié (10).

2. Procédé selon la revendication 1, dans lequel le système de coordonnées sur l'échantillon biologique est un système de coordonnées cartésiennes orthogonal.

3. Procédé selon l'une quelconque des revendications 1 à 2, dans lequel créer la combinaison spatiale de concentrations de fluorophores actifs comprend : colorer (S120) l'échantillon biologique (10) avec ladite pluralité de fluorophores (31, 32, 33, 34).

4. Procédé selon l'une quelconque des revendications 1 à 3, dans lequel de multiples fluorophores différents sont fixés à une molécule unique, par exemple fixés de manière covalente à un oligonucléotide.

5. Procédé selon la revendication 4, dans lequel de multiples fluorophores différents se fixent à des parties du composant biologique avec des rapports constants.

6. Procédé selon l'une quelconque des revendications 3 à 5, comprenant en outre : modifier (S130) les concentrations de fluorophores actifs de certains fluorophores de la pluralité de fluorophores (31, 32, 33, 34) dans l'échantillon biologique (10) après la coloration (S120).

7. Procédé selon l'une quelconque des revendications 1 à 6, comprenant : créer une pluralité de gradients de concentrations de fluorophores actifs dans l'échantillon biologique (10), en particulier la pluralité de gradients de concentrations de fluorophores actifs étant orthogonaux les uns aux autres.

8. Procédé selon la revendication 6 ou 7, dans lequel la modification (S130) desdites concentrations de fluorophores actifs est effectuée par l'un d'un photoblanchiment, d'une photoactivation ou d'une photocommutation, en particulier dans lequel la modification (S212) est effectuée par photoblanchiment à l'aide d'une feuille lumineuse (40) avec des périodes d'exposition différentielles de régions de l'échantillon biologique (10) à la feuille lumineuse (40).

9. Procédé selon la revendication 8, comprenant : mesurer des niveaux de fluorescence d'un échantillon d'étalonnage avant et après la modification des concentrations de fluorophores actifs, pour générer des courbes d'étalonnage de blanchiment (48) qui définissent les paramètres de blanchiment à utiliser pendant la modification du niveau résultant de la concentration de fluorophores actifs dans l'échantillon d'intérêt (10).

10. Procédé selon l'une quelconque des revendications ci-dessus, dans lequel l'encodage optique (S100) est effectué par différents fluorophores (31, 32, 33), qui ont différents spectres d'excitation et différents spectres d'émission définissant au moins trois canaux de fluorescence.

11. Procédé selon l'une quelconque des revendications ci-dessus, comprenant : utiliser des fluorophores supplémentaires (34) en tant que fluorophores de contrôle, en particulier le procédé comprenant en outre : normaliser les niveaux de fluorescence mesurés des fluorophores autres que les fluorophores de contrôle (31, 32, 33) par rapport aux niveaux de fluorescence des fluorophores de contrôle (34).

12. Procédé selon l'une quelconque des revendications ci-dessus, comprenant : mettre en correspondance des niveaux de fluorescence mesurés des différents fluorophores (31, 32, 33) desdits composants biologiques dissociés (12) avec l'espace physique dans l'échantillon biologique (10) avant dissociation, de sorte que chaque combinaison de couleurs mesurée après la dissociation est attribuée à des emplacements probables dans l'espace physique.

13. Procédé selon la revendication 11, dans lequel la mise en correspondance des niveaux mesurés de fluorescence de composants biologiques dissociés (12) avec l'espace physique comprend : imager l'échantillon biologique (10) avant la dissociation et mettre en correspondance l'espace colorimétrique mesuré desdits composants biologiques dissociés avec l'espace colorimétrique mesuré dans le système utilisé pour imager l'échantillon biologique (10) avant dissociation, par exemple au moyen d'un algorithme de correspondance d'histogramme pour chaque canal de fluorescence.

14. Procédé selon la revendication 12 ou 13, dans lequel des échantillons de contrôle colorés par un seul fluorophore sont utilisés pour déterminer la diaphonie entre des combinaisons par paires de canaux, à la fois dans le système utilisé pour imager l'échantillon biologique (10) avant dissociation et dans le système utilisé pour mesurer les signaux desdits composants biologiques (12) après dissociation, pour corriger les données de canal dans les deux systèmes avant la correspondance d'histogramme de chaque canal entre les deux instruments.

15. Procédé selon l'une quelconque des revendications ci-dessus, comprenant en outre : reconstruire l'échantillon biologique (10) à partir de la fluorescence mesurée.

16. Système de détermination d'un emplacement spatial d'un ou de plusieurs composants biologiques (12) dans un échantillon biologique (10), comprenant :
- une unité d'encodage optique (50) prévue pour encoder de manière optique les composants biologiques (12) dans l'échantillon biologique (10) avec une pluralité de fluorophores (31, 32, 33, 34) pour créer une combinaison de concentrations de fluorophores actifs indicative de l'emplacement spatial desdits un ou plusieurs composants biologiques (12), dans lequel l'unité d'encodage optique est adaptée pour encoder de manière optique un système de coordonnées d'échantillon sur l'échantillon biologique, avec la combinaison de concentrations de fluorophores actifs étant, pour les composants biologiques, un ensemble de coordonnées dans ledit système de coordonnées d'échantillon ;
- un système de mesure optique (80) pour mesurer la fluorescence après dissociation, comprenant un parmi un trieur ou analyseur de cellules activées par fluorescence, un dispositif microfluidique
- une unité de mise en correspondance (90), configurée pour mettre en correspondance la fluorescence mesurée à partir desdits composants biologiques dissociés (12), avec l'emplacement spatial desdits composants biologiques (12) dans l'échantillon non dissocié (10), dans lequel l'unité de mise en correspondance (90) est adaptée pour déterminer, pour chaque composant biologique dissocié, l'ensemble de coordonnées dans ledit système de coordonnées d'échantillon, pour obtenir l'emplacement spatial de chacun desdits composants biologiques dans l'échantillon non dissocié (10).

17. Système selon la revendication 16, dans lequel l'unité d'encodage optique comprend une unité d'imagerie pour modifier la distribution spatiale de la pluralité de concentrations de fluorophores et mesurer la distribution spatiale de la pluralité de concentrations de fluorophores après la modification, en particulier dans lequel l'unité d'imagerie comprend un microscope à éclairage sélectif d'un plan ou un autre microscope à feuille lumineuse (50) pour imager l'échantillon biologique (10) avant dissociation.

18. Système selon l'une quelconque des revendications 16 ou 17, dans lequel le système de coordonnées d'échantillon est un système de coordonnées cartésiennes orthogonal.
